# EUROPEAN PATENT APPLICATION

(11) **EP 4 599 838 A1**
(43) Date of publication of application: **13.08.2025**
(21) Application number: 23874076.5
(22) Date of filing: 01.09.2023
(51) Int. Cl.: A61K 36/185, A23L 33/105, A61K 31/7048, A61P 25/28

(54) **ALZHEIMER-TYPE DEMENTIA PREVENTING AGENT, AND METHOD FOR PRODUCING SAME**

(30) Priority: 03.10.2022 JP 2022159550
(71) Applicant: Sakamoto Yakusouen, LLC., Otsu-shi, Shiga 520-0113 (JP)
(72) Inventor: YAMAHARA, Johji, Otsu-shi, Shiga 520-0113 (JP)
(74) Representative: Vossius & Partner Patentanwälte Rechtsanwälte mbB
(86) International application number: PCT/JP2023/032051
(87) International publication number: WO 2024/075453

(57) **Abstract**

The objective of the present invention is to provide an Alzheimer-type dementia suppressant that is safe and thus can be administered daily and utilized as a health food and by which Alzheimer-type dementia can be suppressed, and a method for producing the Alzheimer-type dementia suppressant. In addition, the objective of the present invention is also to provide a use of an aboveground part of Sesamum indicum or an extract thereof for suppressing Alzheimer-type dementia, and a method for suppressing Alzheimer-type dementia. The Alzheimer-type dementia suppressant according to the present invention is characterized in comprising an aboveground part of Sesamum indicum or an extract thereof as an active ingredient.

## Description

### TECHNICAL FIELD

The present invention relates to an Alzheimer-type dementia suppressant that is safe and thus can be administered daily and utilized as a health food and by which Alzheimer-type dementia can be suppressed, and a method for producing the Alzheimer-type dementia suppressant.

### BACKGROUND ART

The prevalence of dementia among people aged 65-69 is 1.5%, but the prevalence of dementia among people aged 85-89 is as high as 44.3%, in accordance with "Prevalence of dementia by age group in a cohort of 10,000 people" (Non-patent document 1) published by the Ministry of Health, Labor and Welfare. In addition, the same trend can be seen in the report described in Non-patent document 2.

The number of patients with Alzheimer-type dementia among dementia is 57 million worldwide in 2019, and is expected to reach 150 million by 2050. Alzheimer-type dementia is induced by the accumulation of amyloid beta (hereafter referred to as "Aβ") in the brain over many years. Thus, it may be important for suppressing Alzheimer-type dementia to inhibit the accumulation of Aβ by eliminating the accumulation of Aβ precursor in early stage. In addition, it is described in Non-patent document 2 that human islet amyloid polypeptide (hereinafter abbreviated as "hIAPP") similar to Aβ accumulates in human pancreatic β cell and is involved in the development of Alzheimer's disease and type 2 diabetes.

An Alzheimer-type dementia drug that is safe, inexpensive, and can be taken easily on a daily basis for many years has been demanded as described above, since Alzheimer-type dementia is indued by the accumulation of Aβ in the brain for many years. For example, grape seed extract, turmeric extract, Uncaria tomentosa extract, green tea extract and Perilla frutescens var. crispa 'Viridi-crispa' extract are respectively described in Patent documents 1 to 5.

### PRIOR ART DOCUMENT

### PATENT DOCUMENT

Patent document 1: WO 2009/137818
Patent document 2: WO 2007/109210
Patent document 3: WO 2002/042429
Patent document 4: WO 2001/049307
Patent document 5: JP 6707251 B

### NON-PATENT DOCUMENT

Non-patent document 1: Ministry of Health, Labor and Welfare, "Trends in Dementia Policy", [online], [searched on August 23, 2022], internet <https://kouseikyoku.mhlw.go.jp/kantoshinetsu/houkatsu/0002378 03.pdf>
Non-patent document 2: SHINOHARA Moeko, Farumasia, 2022, vol. 58, no. 8, p. 768-771

### DISCLOSURE OF THE INVENTION

### PROBLEMS TO BE SOLVED BY THE INVENTION

Substances derived from a plant to inhibit Aβ accumulation have been known as described above. On the one hand, Alzheimer-type dementia drug that is actually on the market in Japan is a donepezil hydrochloride tablet only. But not only the donepezil hydrochloride tablet may be associated with a severe adverse effect but also cannot fundamentally treat Alzheimer-type dementia and merely suppresses the progression of the symptoms. Thus, a safe drug that can fundamentally treat Alzheimer-type dementia, for example, that can suppress the accumulation of Aβ, has been required.

The objective of the present invention is to provide an Alzheimer-type dementia suppressant that is safe and thus can be administered daily and utilized as a health food and by which Alzheimer-type dementia can be suppressed, and a method for producing the Alzheimer-type dementia suppressant. In addition, the objective of the present invention is also to provide a use of an aboveground part of Sesamum indicum or an extract thereof for suppressing Alzheimer-type dementia, and a method for suppressing Alzheimer-type dementia.

### MEANS FOR SOLVING THE PROBLEMS

The inventor of the present invention made extensive studies to solve the above-described problems. As a result, the inventor completed the present invention by finding that a component by which Alzheimer-type dementia can be fundamentally suppressed is contained in an aboveground part of Sesamum indicum, which is safe and useful as food.

Hereinafter, the present invention is described.
[1] An Alzheimer-type dementia suppressant comprising an aboveground part of Sesamum indicum or an extract thereof as an active ingredient.
[2] The Alzheimer-type dementia suppressant according to the above [1], wherein the aboveground part of Sesamum indicum is a leaf.
[3] The Alzheimer-type dementia suppressant according to the above [1] or [2], wherein the aboveground part or the extract thereof comprises pedaliin.
[4] Use of an aboveground part of Sesamum indicum or an extract thereof for suppressing Alzheimer-type dementia.
[5] The use according to the above [4], wherein the aboveground part of Sesamum indicum is a leaf.
[6] The use according to the above [4] or [5], wherein the aboveground part or the extract thereof comprises pedaliin.
[7] A method for suppressing Alzheimer-type dementia, the method comprising the step of administering an aboveground part of Sesamum indicum or an extract thereof as an active ingredient.
[8] The method according to the above [7], wherein the aboveground part of Sesamum indicum is a leaf.
[9] The method according to the above [7] or [8], wherein the aboveground part or the extract thereof comprises pedaliin.
[10] A method for producing an Alzheimer-type dementia suppressant, the method comprising steps of:
   sowing a seed of Sesamum indicum to be grown, and
   collecting an aboveground part of the grown Sesamum indicum.
[11] The method according to the above [10], wherein the aboveground part is collected before the Sesamum indicum has 1 or more buds and a seed is mature.
[12] The method according to the above [10] or [11], further comprising the step of obtaining an extract solution by extracting an active ingredient from the aboveground part of Sesamum indicum with a solvent.
[13] The method according to the above [12], wherein the solvent is an alcohol solvent.
[14] The method according to the above [13], further comprising the step of obtaining an extraction residue by subjecting the extract with the alcohol solvent to an extraction with a hydrocarbon solvent.

### EFFECT OF THE INVENTION

The active ingredient against Alzheimer-type dementia according to the present invention is safe and thus can be taken regularly every day, since the active ingredient is contained in the aboveground part of Sesamum indicum, which is used for food. In addition, the Alzheimer-type dementia suppressant of the present invention may fundamentally prevent and/or treat Alzheimer-type dementia by removing the cause of Alzheimer-type dementia. For example, the active ingredient reduces Aβ precursor and inhibits the accumulation of Aβ. The present invention is, therefore, industrially very excellent as the preventive agent and the therapeutic agent for Alzheimer-type dementia.

### BRIEF DESCRIPTION OF THE DRAWINGS

[Figure 1] Figure 1 is the PCA figure to show the difference between the genome expressions of a normal aged mouse "SAM-R" and a senescence-accelerated model mouse "SAM-P8".
[Figure 2] Figure 2 is the PCA figure to show the difference between the genome expressions of a normal aged mouse "SAM-R" and a senescence-accelerated model mouse "SAM-P8" to which the extract powder derived from the leaf of Gomakurohachi (registered trademark) (SSa) was orally administered for 7 days.
[Figure 3] Figure 3(1) is the figure to show the PCA analysis result in the gene levels between the control group and the donepezil-administered group, and Figure 3(2) is the figure to show the PCA analysis result in the exon levels.
[Figure 4] Figure 4(1) is the figure to show the PCA analysis result in the gene levels between the control group and the acteoside-administered group, and Figure 4(2) is the figure to show the PCA analysis result in the exon levels.
[Figure 5] Figure 5(1) is the figure to show the PCA analysis result between the control group and the SSa extract powder-administered group, and Figure 5(2) is the figure to show the PCA analysis result in the exon levels.

### MODE FOR CARRYING OUT THE INVENTION

The Alzheimer-type dementia suppressant of the present invention comprises an aboveground part of sesame, "Sesamum indicum", or an extract thereof as an active ingredient. The term "active ingredient" means the component that has the function to suppress Alzheimer-type dementia among the components contained in the Alzheimer-type dementia suppressant of the present invention in this disclosure. In other words, the Alzheimer-type dementia suppressant of the present invention comprises an aboveground part of Sesamum indicum or an extract thereof as an active ingredient in an amount to exert the effect to suppress Alzheimer-type dementia. Specifically, the concentration of the aboveground part of Sesamum indicum or the extract thereof in the Alzheimer-type dementia suppressant of the present invention is not particularly restricted, and for example, may be adjusted to 10 mass% or more and 100 mass% or less. The term "suppression" includes the concept of prevention to suppress the symptom onset of Alzheimer-type dementia and the concept of treatment to alleviate and/or remit the symptom of Alzheimer-type dementia in this disclosure.

Sesame, "Sesamum indicum", is an annual grass classified in Pedaliaceae Sesamum, and there are said to be about 3,000 varieties of sesame based on differences in seed figure such as size and color. Though the kind of sesame is not particularly restricted, the present inventor experimentally confirmed that the leaf of Gomakurohachi (registered trademark) has the excellent function to suppress Alzheimer-type dementia.

Gomakurohachi (registered trademark) has been cultured in a specific region of Myanmar in the name of Sa Mong Nan Net Thae and is small seed black sesame. The seed thereof is very similar in weight and appearance to a seed of registered lignan sesame. The registration number of the lignan sesame is 19697, the application number is 20790, the kind of agricultural, forest, or aquatic plant is Sesamum indicum L., and the name of breed variety is ITCFA2001; and the registration number is 19698, the application number is 20791, the kind of agricultural, forest, or aquatic plant is Sesamum indicum L., and the name of breed variety is ITCFA2002. The present inventor found out that the above sesame varieties are evolutionarily different from each other by the genomic analysis result. For example, the inventor found the large insertion and deletion between the two genomes. The difference between the genomes of Gomakurohachi and the lignan sesame can be verified by PCR.

The aboveground part means the part that is not an underground part and can be seen above ground in the case where a sesame seed is sown and grown. The aboveground part of sesame can be obtained by cutting and removing the underground part from a cultivated sesame.

For example, the aboveground part of sesame is preferably an above ground part of stubble having 2 or more true leaves. In addition, the stubble before a ripe seed is born, such as stubble having an unripe fruit, is preferably collected as the aboveground part. Furthermore, the stubble of which height is 50 cm or more and 200 cm or less, the stubble having 1 or more buds before blossoming, and the stubble having 1 or more flowers may be also collected. The period from sowing to collecting may be appropriately adjusted. For example, the period may be adjusted to 1 month or more, is preferably 2 months or more or 2.5 months or more, and may be 5 months or less, is preferably 4 months or less, more preferably 3.5 months or less.

The aboveground part of sesame is preferably a leaf. The leaf may have a leafstalk or may not have a leafstalk. A leaf not having a leafstalk has a larger content of the active ingredient per weight, but a leaf having a leafstalk has the advantage of being less labor-intensive to collect.

The aboveground part of sesame may be directly used. Alternatively, for example, the aboveground part may be subjected to 1 or more treatments selected from water washing, drying, pulverization, hydrolysis and extraction. For example, the aboveground part may be treated at 25±5°C under a humidity of 90% or more and 100% or less for 1 hour or more and 5 hours or less for a hydrolysis treatment, or the above ground part may be rotated in a drum and then heated for stopping hydrolysis, as a hydrolysis treatment. Bitterness may be reduced by a hydrolysis treatment.

The form of the active ingredient of the Alzheimer-type dementia suppressant according to the present invention is not particularly restricted, and an example thereof includes liquid, paste and powder. An example of the powder includes coarse powder, micro powder and nano micro powder.

The form of the Alzheimer-type dementia suppressant according to the present invention is not particularly restricted, and an example thereof includes solid, gel and liquid. More specifically, an example of the form includes powder medicine, fine grain agent, granule, tablet, coated tablet, capsule, lozenge and liquid medicine.

The Alzheimer-type dementia suppressant of the present invention may contain an additive in addition to the active ingredient depending on the dosage form thereof. An example such an additive includes excipient, binder, lubricant, disintegrant, emulsifier, stabilizer, absorption promoter, fractionalization agent and preservative. The content amount of the additive is not particularly restricted and may be appropriately adjusted.

For example, the Alzheimer-type dementia suppressant of the present invention can be produced by the method comprising the following steps.

### 1. Step to sow and grow sesame

A sesame seed is sown to be grown in this step. This step may be carried out in accordance with the general way of growing sesame. For example, a sesame seed may be sown after maximum temperature exceeds 20°C and may be sown from mid-May to mid-June in Japan. Specifically, about 1 cm-deep hole is dug, 5 to 6 seeds are added into 1 hole, and then the seeds are covered with soil and sufficient amount of water is given. When sesame has 1 to 2 true leaves, a weaker sesame is thinned so that there are about 3 plants per 1 spot. When sesame has 3 to 4 true leaves, a weaker sesame is thinned so that there are 2 plants per 1 spot. When sesame has 5 to 6 true leaves, a weaker sesame is thinned so that there is 1 plant per 1 spot.

### 2. Step to collect aboveground part of sesame

The aboveground part of grown sesame is collected in this step. Specifically, the aboveground part may be separated from the underground part of grown sesame. The time for collection and the aftertreatment are described above.

### 3. Step of extraction

The active ingredient is extracted from the aboveground part of sesame using a solvent in this step. This step may be carried out or may not be carried out. The function of the active ingredient to suppress Alzheimer-type dementia may be further improved by extraction.

The solvent to be used for extraction in this step is not particularly restricted and may be appropriately selected. For example, an example of the solvent includes an aqueous solvent in terms of safety. Such an aqueous solvent means water or a mixed solvent of water and a water-soluble organic solvent. The rate of a water-soluble organic solvent in the mixed solvent may be appropriately adjusted, and for example, may be adjusted to 20 mass% or more. The rate is preferably 30 mass% or more or 40 mass% or more, and more preferably 50 mass% or more. The upper limit of the rate is not particularly restricted, and for example, the rate is preferably 98 mass% or less or 95 mass% or less, and more preferably 90 mass% or less or 80 mass% or less. An example of the aqueous organic solvent includes an alcohol solvent such as methanol, ethanol and 2-propanol; an ether solvent such as tetrahydrofuran; an amide solvent such as dimethylformamide and dimethylacetamide; and a sulfoxide solvent such as dimethylsulfoxide. Ethanol is preferred as the aqueous organic solvent, since ethanol is relatively harmless to a human body. The kind of water is not particularly restricted, and purified water, distilled water, pure water, tap water and the like can be used without restriction.

The extraction condition in this step is not particularly restricted, and an ordinary method can be used. For example, about 5 mL or more and about 100 mL or less of a solvent is added per 1 g of the aboveground part of sesame for extraction for about 30 minutes or more and about 10 hours or less. The extraction temperature is also not particularly restricted. The active ingredient may be extracted at atmospheric temperature or the boiling point of the solvent or lower, or the mixture may be heated to reflux. For example, when water is used as a solvent, the mixture may be heated at 60°C or higher and 100°C or lower.

A general aftertreatment may be carried out after the extraction. For example, a solid content may be removed from the mixture after the extraction by filtration or centrifugation. In addition, the thus obtained solution may be subjected to distillation, concentration or drying. An example of a drying method includes drying by heating, drying under reduced pressure, lyophilization and spray drying. Furthermore, the active ingredient may be further purified by chromatography or the like.

The method for suppressing Alzheimer-type dementia according to the present invention comprises the step of administering an aboveground part of sesame, "Sesamum indicum", or an extract thereof as an active ingredient.

The usage amount of the Alzheimer-type dementia suppressant according to the present invention is not particularly restricted and may be appropriately adjusted depending on whether the suppressant is used preventively or therapeutically, or the severity, other condition, age and sex of a patient. For example, when the Alzheimer-type dementia suppressant of the present invention is orally taken, 10 mg or more of the suppressant may be used in solid content equivalent. When the suppressant is externally applied, 100 mg or more of the suppressant may be used in solid content equivalent. Since the Alzheimer-type dementia suppressant of the present invention is very safe, the upper limit of the usage amount is not particularly restricted. For example, the usage amount can be adjusted to 2 g or less in the case of internal use and 5 g or less in the case of external use, per 1 day in solid content equivalent. The number of use of the suppressant per 1 day can be adjusted to about 1 time or more and about 5 times or less.

The Alzheimer-type dementia suppressant of the present invention can be administered to not only a human but also an animal other than a human. An example of the object animal includes livestock such as horse, cow, pig, sheep, goat, camel and llama; a competitive animal such as racehorse; a pet such as dog and cat; an experimental animal such as mouse, rat, guinea pig and rabbit; poultry such as chicken, duck, turkey and ostrich; and fish and shellfish, such as cultured fish.

The Alzheimer-type dementia suppressant of the present invention has the very excellent function to suppress an Aβ aggregation. Specifically, the suppressant has the functions to reduce an Aβ precursor, eventually suppress the generation of an Aβ accumulation body, and normalize gene expression. The Alzheimer-type dementia suppressant of the present invention is therefore useful as a preventive agent and/or a therapeutic agent of Alzheimer-type dementia.

The present application claims the benefit of the priority date of Japanese patent application No. 2022-159550 filed on October 3, 2022. All of the contents of the Japanese patent application No. 2022-159550 filed on October 3, 2022, are incorporated by reference herein.

### EXAMPLES

Hereinafter, the present invention is described in more detail with Examples. The present invention is however not restricted to the following Examples in any way, and it is possible to work the present invention according to the Examples with an additional appropriate change within the range of the above descriptions and the following descriptions. Such a changed embodiment is also included in the technical scope of the present invention.

### Example 1: Production of SSa extract powder

The seed of Gomakurohachi (registered trademark) was sown in April, 2019. When a bud was observed after about 3 months, the leaves were collected and dried at 60°C for 12 hours. The dried leaves (100 g) were added to 60% ethanol water solution (1 L), and the mixture was shaken up well and stood still at the atmospheric temperature overnight. Then, the solid content was removed by filtration to obtain an extract solution. The thus obtained extract solution was concentrated at 60°C under reduced pressure to obtain an extract powder (amount: 15.5 g). Hereinafter, the obtained powder is referred to as "SSa extract powder".

### Example 2: Animal experiment

Seven-week-old male normal aged mouse SAM-R and male senescence-accelerated model mouse SAM-P8 that were respectively obtained from Japan SLC were arbitrarily divided into two groups of 10 mice each and acclimatized for 1 week. Then, 500 mg/kg of SSa extract powder was orally administered to SAM-R SSa extract powder-administered group and SAM-P8 SSa extract powder-administered group for 7 days. Each group of mice other than the above groups was given normal feed and water freely. Next, after the mice were reared for 30 days, the mice were humanely killed by anesthesia. The whole brain was taken out and preserved at -80°C by the genome analysis.

The reagent for dissolving lipid and tissue ("QIAzol Lysis Reagent" manufactured by QIAGEN) was added to the brain sample (100 to 150 mg), and the brain sample was pulverized using a homogenizer ("POLYTRON" manufactured by CENTRAL SCIENTIFIC COMMERCE) for 20 to 40 seconds and then stood still at room temperature for 5 minutes to be dissolved. Next, chloroform (0.2 mL) was added thereto, and the mixture was stirred well for 15 seconds and then stood still for 2 to 3 minutes. The mixture was separated into a water layer, a middle layer and a phenol-chloroform layer by centrifugation at 4°C and 13000 g for 15 minutes. Since RNA was contained in the water layer, the water layer was transferred into another tube. Isopropyl alcohol (0.5 mL) was added thereto, and the mixture was stirred and then stood still at room temperature for 10 minutes. The mixture was further centrifuged at 4°C and 13000 g for 15 minutes, the supernatant was removed, and 70% ethanol (1 mL) was added to the precipitation. After the mixture was stirred well, the mixture was centrifuged at 4°C and 13000 g for 5 minutes and the supernatant was removed. The precipitation was dried in air for 5 to 10 minutes to vaporize ethanol. Pure water (50 µL) was added thereto, and the mixture was preserved at -80°C by the RNA sequencing.

The specific mRNA was concentrated from the obtained total RNA using an RNA sample preparation kit ("TruSeq Standard mRNA Library Prep kit" manufactured by Illumina). The mRNA were used as a template to synthesize cDNA, and a library for sequencing was prepared. Then, the sequences of the cDNA library were determined using a next-generation DNA sequencer ("NoVaSeq6000" manufactured by Illumina) in the conditions of 100 bp, Paired-end and 40,000,000 read per 1 sample.

STAR>cufflinks>cuffdif analysis was carried out to compare the analysis results. GRCm38.92 was used as a mouse genome database, and the gene expression amount in gene level and in exon level as the component element of the gene was analyzed. Specifically, a reference mouse sequence, "GRCm38.92", was mapped using a mapping tool referred to as STAR and further assembled using Cufflinks to count the mapped reads in gene unit and exon unit. In addition, the amount of the expressed gene was determined using cuffdiff, and the genes/exon site in which the expression amounts were changed between 2 groups was specified.

Next, PCA: Principal Component Analysis was carried out, and the difference of genome expression amounts was plotted on principal axes, "PC1 axis and PC2 axis". Contribution rates (eigen vector) under PC1 axis and PC2 axis are important in such a case, and 2 groups can be compared on the basis of the axis having high contribution rate.

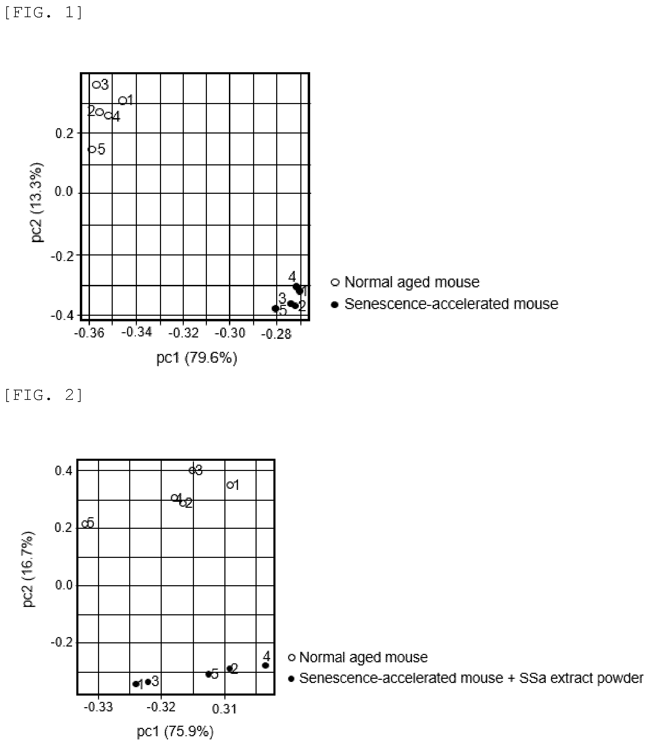

Figure 1 is the PCA figure to show the difference between the gene expression amounts of a normal aged mouse SAM-R and a senescence-accelerated model mouse SAM-P8.

It can be found in Figure 1 that there is also large difference between the gene expressions of a normal aged mouse SAM-R and a senescence-accelerated model mouse SAM-P8 on the horizontal axis (PC1 axis), which contributes to a contribution rate (eigen vector) more greatly.

Figure 2 is the PCA figure to show the difference between the genome expressions of a normal aged mouse SAM-R and a senescence-accelerated model mouse SAM-P8 to which the SSa extract powder was orally administered for 7 days.

It can be found in Figure 2 that the genome expression in the case where the SSa extract powder was administered to senescence-accelerated model mouse SAM-P8 was close to the genome expression of normal aged mouse SAM-R on the horizontal axis (PC1 axis), which contributes to a contribution rate (eigen vector) more greatly.

### Example 3: Animal experiment

Seven-week-old male senescence-accelerated model mouse SAM-P8 obtained from Japan SLC was randomly divided into 4 groups of 10 mice each of a control group, a donepezil-administered group, an acteoside-administered group and an SSa extract powder-administered group. Donepezil is a commercially available Alzheimer's drug, and the trade name of donepezil is Aricept manufactured by Eisai. Acteoside is manufactured by Nanjing Spring & Autumn Biological Engineering.

Donepezil in a dose of 5 mg/kg, acteoside in a dose of 100 mg/kg and the SSa extract powder in a dose of 500 mg/kg were continuously orally administered to each group for 7 days. Each group of mice other than the above groups was given normal feed and water freely. Next, after the mice were reared for 30 days, the mice were humanely killed by anesthesia. The whole brain was taken out and preserved at -80°C by the genome analysis. Then, PCA analysis in a gene level and an exon level was carried out similarly to Example 2.

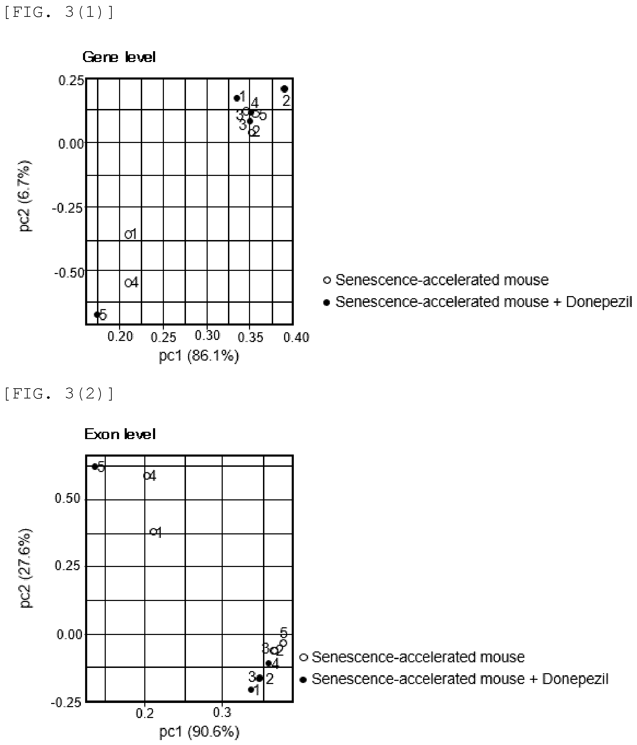

The PCA analysis results between the control group and the donepezil-administered group, the acteoside-administered group or the SSa extract powder-administered group are respectively demonstrated in Figures 3 to 5. The upper (1) shows the PCA analysis result in a gene level and the lower (2) shows the PCA analysis result in an exon level in Figures 3 to 5.

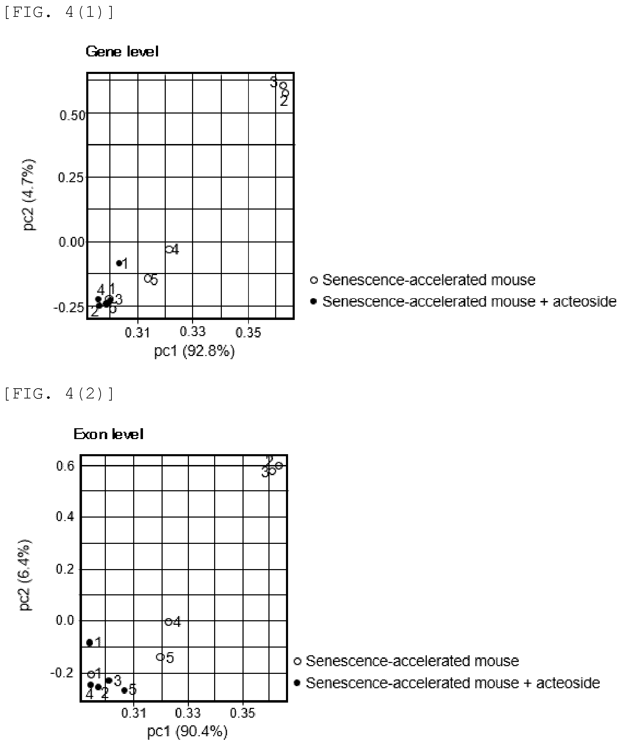

It can be found in Figures 3 and 4 that there was not a large difference between the expression patterns of the control group and the donepezil-administered group or the acteoside-administered group on the horizontal axis (PC1 axis), which contributes to a contribution rate (eigen vector) more greatly.

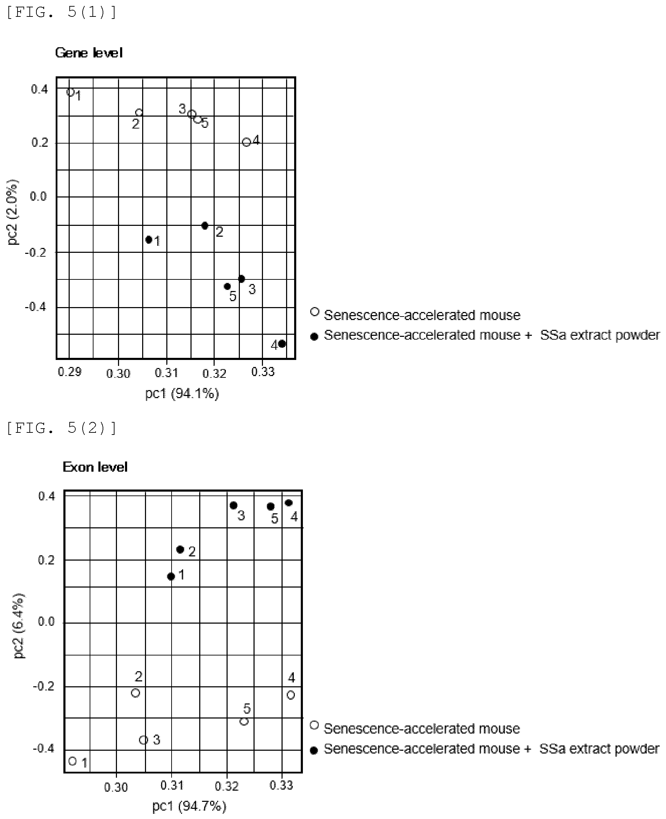

On the one hand, it can be found in Figure 5 that there was a significant difference between the expression patterns of the control group and the SSa extract powder-administered group on the horizontal axis (PC1 axis), which contributes to a contribution rate (eigen vector) more greatly. In addition, the expression pattern thereof was close to the expression pattern of the normal aged mouse shown in Figure 2.

In other words, though donepezil and acteoside may reduce the accumulation of Aβ and relieve the symptom after the accumulation of Aβ and the onset of dementia, the expression pattern of the genome in the brain may be normalized by absorbing some kind of the components derived from the SSa extract powder of the present invention to affect the gene expression.

### Example 4: Clinical experiment

A single-center placebo-controlled randomized single blind parallel-group trial concerning the function of SSa to reduce an amyloid β precursor in blood plasma was carried out under the approval of the ethics committee in the following condition.

Twelve apparently healthy elderly subjects aged 65 years or older (mean age: 68.75 years) were divided into two groups including 3 males and 3 females each, a mulukhiya powder-administered group as a placebo group and an SSa extract powder-administered group. Since the SSa extract powder had the useful effect on the genome expression in the brain of the senescence-accelerated model mouse SAM-P8 by administering the SSa extract powder in a dose of 500 m/kg in Example 3, the inventor considered that a human needs to take 3 to 4 g/day.

The SSa extract powder was administered to the SSa extract powder-administered group 2 times per 1 day in the morning and the evening in a dose of 1.5 g per 1 time for 2 months. The mulukhiya powder was administered to the placebo group 2 times per 1 day in the morning and the evening in a dose of 1.5 g per 1 time for 2 months.

The blood was collected 2 times in the morning of the test start date before starting the test and 4 to 5 hours after the last administration, and the amount of an amyloid β (Aβ) precursor in the blood was measured by IP-MS (JP 6410810 B; Akinori Nakamura et al., Nature, 554, 249-254, 2018), in which immunoprecipitation method and mass spectrometry were combined. Specifically, the collected blood was treated with EDTA and then centrifuged at 3,000 rpm. The separated blood plasma was preserved at -77 to -79°C and subjected to the measurement of an amyloid β precursor. The biomarker index to show the cumulative amount of an amyloid β precursor is shown in Table 1.

**Table 1**

| | Before administration | After administration | Difference | Average |
|---|---|---|---|---|
| SSa extraction powder-administered group | 0.852 | -0.406 | -1.258 | -1.207 ±0.2244 |
| | 1.220 | 0.016 | -1.204 | |
| | 1.808 | -1.134 | -2.942 | |
| | 0.527 | -0.445 | -0.972 | |
| | -1.008 | -1.388 | -0.38 | |
| | -0.471 | -0.957 | -0.486 | |
| Placebo group Mulukhiya powder-administered group | -0.366 | -0.898 | -0.532 | -0.4925 ±0.2244 |
| | 1.849 | 1.579 | -0.27 | |
| | 0.191 | -0.883 | -1.074 | |
| | 2.039 | 0.837 | -1.202 | |
| | -0.109 | -0.095 | 0.014 | |

The rate of the subject of which biomarker index before the administration of the sample was 1 or more was 33.3% as 2/6 in both of the SSa extract powder-administered group and the placebo group as the result shown in Table 1.

The average value of the differences of the biomarker values between "before the administration in the SSa extract powder-administered group" and "after the administration in the SSa extract powder-administered group" was -1.207, and the average value of the differences of the biomarker values between "before the administration in the placebo group" and "after the administration in the placebo group" was -0.4925. There was a significant tendency in the difference between the amounts of an amyloid β precursor of the groups with a risk rate of 0.05 < p < 0.1 in accordance with t-test. In other words, it was found that an amyloid β precursor in blood was significantly reduced by administering the SSa extract powder in comparison with the placebo group.

### Example 5: Component analysis

### (1) Fractionation and Aβ accumulation inhibitory activity

Dried SSa leaves (500 g) collected in Hanase of Kyoto in 2019 were roughly cut and immersed in n-hexane (1 L) at room temperature for 2 hours. The solid content was separated by filtration. The immersion in n-hexane and the separation of the solid content were repeated. The thus obtained filtrate is referred to as the n-hexane fraction. Then, 80% ethanol water solution (1 L) was added to the separated solid content, and the mixture was sometimes stirred at room temperature for 2 hours. The solid content was separated by filtration. The immersion in 80% ethanol water solution and the separation of the solid content were repeated. The thus obtained filtrate was concentrated under reduced pressure to 150 mL.

Chlorophyll was removed by supplying the obtained concentrated 80% ethanol water solution SSa extract (150 mL) to an adsorption column ("DIAION (registered trademark) HP-20" manufactured by Mitsubishi Chemical) and eluting with methanol. The methanol eluate was concentrated under reduced pressure, and ethyl acetate was added to the residue. The mixture was filtrated, and the filtrate was concentrated under reduced pressure to obtain the ethyl acetate fraction. The n-butanol fraction and the water fraction were similarly obtained except that n-butanol and water were used. Each fraction was concentrated under reduced pressure.

The Aβ accumulation inhibitory activities of each fraction were determined using Thioflavin-T in the following condition. Aβ derived from human ("Human, 1-42" manufactured by Peptide Institute) was dissolved in 0.1% ammonia water in a concentration of 250 µM. Each of the fraction samples was added to a phosphate buffer (0.05 M sodium phosphate + 0.1 M sodium chloride, pH7.4) (hereinafter abbreviated as "PBS") in the final concentrations of 0.01 g/L, 0.05 g/L and 0.1 g/L. The Aβ solution was added thereto in the final concentration of 25 µM for the reaction at 37°C. A part of the reaction mixture was collected 0, 4, 8 and 24 hours after the start of the reaction, and 2.5 µL of the collected reaction mixture was added to each well of a 96 well plate for fluorescence measurement ("Nunc FluoroNunc plate" manufactured by Thermo Fisher Science). Then, Thioflavin-T solution was dissolved in 50 mM glycine-sodium hydroxide buffer (pH 8.5), and the solution (250 µL) was added to each well. The fluorescence intensity emitted when the β sheet of the Aβ fiber was labeled with Thioflavin-T was measured. The fluorescence intensity was measured using a microplate reader ("Wallac 1420 ARVO MX" manufactured by Perkin Elmer) in the condition of a wavelength of Excitation: 420 nm and Emission: 485 nm. The IC₅₀ value was calculated using the statistical analysis software PriProbit from the inhibitory rates in the cases of each sample concentration of 0.01, 0.05, and 0.1 g/L on the basis of the fluorescence intensity after 24 hours on the proviso that the fluorescence intensity of Aβ was regarded as 100. The result is shown in Table 2.

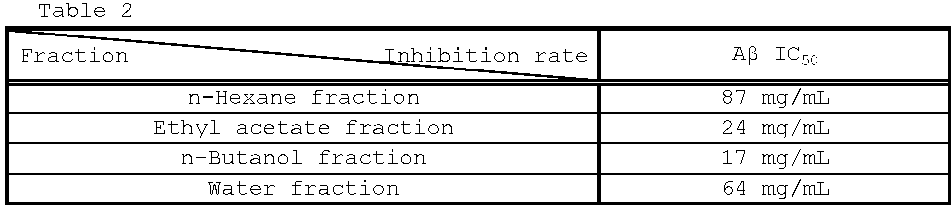

It was found from the result shown in Table 2 that the n-butanol fraction of SSa mainly has the activity to inhibit the accumulation of Aβ protein.

### (2) Aβ accumulation inhibitory activity component and hIAPP accumulation inhibitory activity component

Acteoside is contained in Chorogi (Chinese artichoke) or the like. Chorogi is a labiatae plant, and the tuber part is used for a traditional New Year's food. It has been known that acteoside has Aβ accumulation inhibitory activity (JP 6424757 B; Manami Kurisu et al., Bioscience, Biotechnology, and Biochemistry, 77, 1329-1332, 2013). Thus, the Aβ accumulation inhibitory activities of acteoside and pedaliin as a flavonoid were compared similarly to Example 5(1). In addition, human islet amyloid polypeptide (hIAPP) is an amyloidogenic protein deposit component in islet of Langerhans and is derived from an amyloid precursor protein, and the sequence thereof is similar to that of Aβ. Thus, the hIAPP accumulation inhibitory activity was also tested. Specifically, each compound was added to PBS (0.05 M NaH₂PO₄, 0.1 M NaCl, pH7.4) at the final concentration of 1 µM, 10 µM and 100 µM, and the hIAPP accumulation inhibitory activity was measured similarly to Example 5(1) except that hIAPP manufactured by Karebay Biochem was used in place of Aβ. The result is shown in Table 3.

**Table 3**

| | Aβ IC₅₀ | hIAPP IC₅₀ |
|---|---|---|
| Pedaliin | 2.8 µM | 4.2 µM |
| Acteoside | 13.0 µM | 21.0 µM |

It was found from the result shown in Table 3 that pedaliin has stronger Aβ accumulation inhibitory activity than acteoside, of which Aβ accumulation inhibitory activity has been known. In addition, the hIAPP accumulation inhibitory activity of pedaliin was also stronger than that of acteoside.

### (3) Investigation of preferred time to collect SSa

Gomakurohachi (registered trademark) grown in Hanase was picked at the time of A: plant length was 50 to 60 cm, B: the buds started to form or C: unripe fruit formed. The leaves containing a leafstalk were dried and finely pulverized. The dried pulverized leaves (0.4 g) was added to methanol (500 mL), and the mixture was sometimes stirred for 12 hours for extraction. The mixture was filtrated and then the filtrate was concentrated and dried using a rotary evaporator. The thus obtained residue was dissolved in methanol (1 mL), and the solution was analyzed by LC/MS in the following conditions.

### LC condition

Column: ODS ACQUITY UPLC BEH c18 1.7·m 2.1×100 mm (manufactured by Waters)
Moving phase: A (acetonitrile containing 0.1% formic acid) : B (H₂O containing 0.1% formic acid) = 5 : 95 at first → A : B = 65 : 35 8 min → B = 100 10min → A : B = 5 : 95 15 min
Flow rate: 0.3 mL/min

### MS condition

ESI TOF ms Positive ion mode
Capillary voltage: 3.2 kV
Cone voltage: 20 eV
Source temperature: 120°C

Each sample was injected in an amount of 4 µL and the MS ion of pedaliin was detected to calculate relative composition percentage. The result is shown in Table 4.

**Table 4**

| Collection time | Pedaliin |
|---|---|
| A: Plant height was 50-60 cm | 30% |
| B: Bud term | 32% |
| C: Unripe fruit term | 38% |

It was found from the result shown in Table 4 that the leaves having a leafstalk from the beginning of the bud term to the time when unripe fruit is produced contains a large amount of pedaliin having an excellent Aβ accumulation inhibitory activity and an excellent hIAPP accumulation inhibitory activity. The inventor therefore decided to reap Gomakurohachi (registered trademark) from the time when a bud can be seen to the time when unripe seed can be seen for the Alzheimer-type dementia suppressant.

### Example 6: Genome analysis

The seed of Gomakurohachi (registered trademark) is very similar to the seed of so-called lignan sesame in terms of color and the seed number per 1 g. Thus, the DNA samples were prepared from both of the sesame for genome analysis.

Since it is known that the base number of sesame genome is about 270,000,000 and the inventor obtained sequence data about 55 times as much as the base number of the sesame genome per 1 breed variety in this genome analysis, the inventor could have analyzed the genome region 55 times. Thus, the inventor probabilistically analyzed all of the genome region, and it could be judged that the misreading had been corrected by the analysis program.

It was found from the genome analysis result that Gomakurohachi (registered trademark) is evolutionarily different from lignan sesame, since there are large insertions and deletions between the genome sequences of Gomakurohachi (registered trademark) and lignan sesame. The difference in the genomes of Gomakurohachi and lignan sesame can be verified using PCR in accordance with the obtained analysis data.

## Claims

1. An Alzheimer-type dementia suppressant comprising an aboveground part of Sesamum indicum or an extract thereof as an active ingredient.

2. The Alzheimer-type dementia suppressant according to claim 1, wherein the aboveground part of Sesamum indicum is a leaf.

3. The Alzheimer-type dementia suppressant according to claim 1, wherein the aboveground part or the extract thereof comprises pedaliin.

4. Use of an aboveground part of Sesamum indicum or an extract thereof for suppressing Alzheimer-type dementia.

5. The use according to claim 4, wherein the aboveground part of Sesamum indicum is a leaf.

6. The use according to claim 4, wherein the aboveground part or the extract thereof comprises pedaliin.

7. A method for suppressing Alzheimer-type dementia, the method comprising the step of administering an aboveground part of Sesamum indicum or an extract thereof as an active ingredient.

8. The method according to claim 7, wherein the aboveground part of Sesamum indicum is a leaf.

9. The method according to claim 7, wherein the aboveground part or the extract thereof comprises pedaliin.

10. A method for producing an Alzheimer-type dementia suppressant, the method comprising steps of:
sowing a seed of Sesamum indicum to be grown, and
collecting an aboveground part of the grown Sesamum indicum.

11. The method according to claim 10, wherein the aboveground part is collected before the Sesamum indicum has 1 or more buds and a seed is mature.

12. The method according to claim 10, further comprising the step of obtaining an extract solution by extracting an active ingredient from the aboveground part of Sesamum indicum with a solvent.

13. The method according to claim 12, wherein the solvent is an alcohol solvent.

14. The method according to claim 13, further comprising the step of obtaining an extraction residue by subjecting the extract with the alcohol solvent to an extraction with a hydrocarbon solvent.
